# EUROPEAN PATENT APPLICATION

(11) **EP 3 425 043 A1**
(43) Date of publication of application: **09.01.2019**
(21) Application number: 17759832.3
(22) Date of filing: 24.02.2017
(51) Int. Cl.: C12N 5/07, C12Q 1/02, C12M 3/00

(54) **REGULARLY ARRANGED SPHEROIDS HAVING UNIFORM SIZE, AND USE THEREOF**

(30) Priority: 29.02.2016 JP 2016037519
(71) Applicant: Yonemitsu, Yoshikazu, Fukuoka-shi, Fukuoka 812-8581 (JP); GAIA BioMedicine Inc., Chuo-ku Fukuoka-shi Fukuoka 810-0023 (JP)
(72) Inventor: YONEMITSU, Yoshikazu, Fukuoka-shi Fukuoka 812-8581 (JP); HARADA, Yui, Fukuoka-shi Fukuoka 812-8581 (JP); MORODOMI, Yosuke, Fukuoka-shi Fukuoka 812-8581 (JP); TERAISHI, Koji, Fukuoka-shi Fukuoka 812-8581 (JP)
(74) Representative: Vossius & Partner Patentanwälte Rechtsanwälte mbB
(86) International application number: PCT/JP2017/007029
(87) International publication number: WO 2017/150366

(57) **Abstract**

The object is to provide a screening method for evaluating a large number of candidate compounds with sufficient accuracy, which uses aligned spheroids having equal sizes. The object is achieved by a method for screening for a substance that acts on spheroid formation, which comprises the following steps: (1) the step of inoculating cells on a plate on which a plurality of wells are regularly arranged (well plate), wherein each of the wells has a lowly adsorptive bottom having a U-shaped section, at a density effective for formation of spheroid, and culturing the cells in the plurality of the wells; (2) the step of contacting the cells with a test substance; and (3) the step of observing whether the cells contacted with the test substance form a spheroid or not, and evaluating action of the test substance on spheroid formation on the basis of the observation result as an index.

## Description

### Technical Field

The present invention relates to a culture containing a plurality of aligned spheroids having equal sizes, and use thereof. The culture of the present invention can be used for screening for a compound that inhibits formation of spheroids, and so forth.

### Background Art

Spheroids have an important possibility for use as an in vitro model for examining toxicity of a compound of various concentrations. Use of spheroids in an in vitro model as a reproducible and reliable index of toxicity is a desirable alternative of use of live animals. It is also known that spheroids having a three-dimensional structure may reflect in vivo behaviors of many cells better compared with two-dimensional culture, and researches concerning interaction of tumor and immunocytes using spheroids, screening for drug development using spheroids, and so forth have been reported.

Methods for screening for various drugs using spheroids, and so forth have been examined. For example, Patent document 1 discloses an analysis method comprising exposing spheroids to a compound to be analyzed, and observing whether inhibition of spheroid cell proliferation takes place. Patent document 2 discloses a method for screening for a substance that acts on maintenance of epithelial properties of cells, which comprises the step of culturing cells on a culture base material on which spheroids can be formed, the step of contacting the cells with a test substance, and the step of evaluating effect of the test substance on maintenance of epithelial properties of the cells on the basis of morphological change of spheroids as an index. This reference describes that the cells are cultured on a culture base material having a predetermined uneven structure that functions as a cell adhesion surface. Further, Patent document 3 discloses a method for screening for a compound, which comprises judging whether a test compound has toxicity to hepatocytes on the basis of survival rate of spheroids. Various methods for producing a three-dimensional structure of cells, of which typical example is spheroids, have also been examined (for example, Patent document 4). Use of such three-dimensional structures for experiments such as screening have also been examined.

### Prior Art References

### Patent documents

Patent document 1: International Patent Publication WO2003/058251 (Japanese Patent Unexamined Publication (KOHYO) No. 2005-514042)
Patent document 2: International Patent Publication WO2014/038025 (Japanese Patent Unexamined Publication (KOKAI) No. 2015-33384)
Patent document 3: Japanese Patent Unexamined Publication (KOKAI) No. 2014-79227
Patent document 4: International Patent Publication WO2008/123614 (Japanese Patent No. 4517125)

### Summary of the Invention

### Object to be Achieved by the Invention

When screening for drugs is conducted by using spheroids, it is necessary to evaluate a large number of candidate compounds with sufficient accuracy. Although homogeneous spheroids are required for such a purpose, the conventional spheroid formation methods are insufficient in this respect.

By the way, formation of spheroids by cancer cells is involved in various highly malignant cancers of which distant metastasis is concerned, such as peritoneal dissemination of digestive system cancers. It is thought that inhibition of the formation of spheroids is important for elucidation of mechanisms of such cancers and development of innovative therapeutic techniques for such cancers.

### Means for Achieving the Object

The inventors of the present invention have examined various techniques for preparing spheroids effective for carrying out reliable screening, and effective for elucidation of mechanisms of diseases or conditions in which formation of spheroids is involved, such as peritoneal dissemination, as well as development of therapies of such diseases or conditions. As a result, they found that a plurality of regularly arranged spheroids having equal sizes can be obtained by culturing cells that can form a spheroid under specific conditions. They also found that a screening method using such homogeneous spheroids can actually be carried out with sufficient accuracy, and accomplished the present invention.

The subject matter of the present invention includes the followings.

[1] A culture of cells supported by a plate on which a plurality of wells are regularly arranged (well plate), wherein each of the wells contains not more than one of spheroid.
[2] The culture according to 1, wherein the spheroids in the wells have equal sizes.
[3] The culture according to 1 or 2, wherein each of the wells has a lowly adsorptive bottom having a U-shaped section.
[4] The culture according to any one of 1 to 3, wherein the well plate has 96, 384, or 1536 wells.
[5] The culture according to any one of 1 to 4, wherein the cells are cancerous cells.
[6] The culture according to any one of 1 to 5, which is for use in screening for a compound.
[7] The culture according to 6, wherein the screening is for selecting a candidate compound of anticancer agent.
[8] The culture according to any one of 1 to 5, which is for use in elucidation of a mechanism of a condition or disease in which formation of spheroids is involved.
[9] The culture according to 8, wherein the condition or disease in which formation of spheroids is involved is peritoneal dissemination.
[10] A method for screening for a substance that acts on spheroid formation, which comprises the following steps:
   (1) the step of inoculating cells on a plate on which a plurality of wells are regularly arranged (well plate), wherein each of the wells has a lowly adsorptive bottom having a U-shaped section, at a density effective for formation of spheroid, and culturing the cells in the plurality of the wells;
   (2) the step of contacting the cells with a test substance; and
   (3) the step of observing whether the cells contacted with the test substance form a spheroid or not, and evaluating action of the test substance on spheroid formation on the basis of the obtained observation result as an index.
[11] The screening method according to 10, wherein the cells are cancerous cells.
[12] The screening method according to 10 or 11, wherein the density effective for formation of spheroid is 1.0 x 10⁴ to 3.0 x 10⁴ cells/mL.
[13] A method for producing a plurality of regularly arranged spheroids having equal sizes, which comprises the following steps:
   (1) the step of inoculating cells on a plate on which a plurality of wells are regularly arranged (well plate), wherein each of the wells has a lowly adsorptive bottom having a U-shaped section, at a density effective for formation of spheroid; and
   (2) the step of culturing the cells in the plurality of the wells.
[14] The production method according to 13, wherein the cells are cancerous cells.

### Effect of the Invention

According to the present invention, reliable screening results can be obtained. The Z' value, which is used as an index for measuring optimality of screening conditions and accuracy, is calculated in accordance with the following equation. Z' = 1 - (3SD of high control + 3SD of low control)/(mean of high control - mean of low control)

In the optimization, a Z' value exceeding 0.5 is usually targeted, and in high throughput screening (HTS), the Z' value is calculated for every microtiter plate, and if the Z' value is smaller than 0.5, it is judged that accuracy of assay performed on that microtiter plate is insufficient (Zhang. J.H. et al., J. Biomol. Screen., 4, 67-73 (1999)). According to the present invention, a highly accurate screening can be performed with a Z' value exceeding 0.5.

### Brief Description of the Drawings

[Fig. 1] An example of preparation of regularly arranged spheroids having equal sizes using a plate having 384 wells with U-shaped bottom section. The row indicated with the arrow showed no spheroid formation due to addition of EDTA. In the two rows of the right and left ends, no cell was inoculated.
[Fig. 2] An enlarged photograph of the portion boxed in Fig. 1.
[Fig. 3] Examples of morphology of the spheroids prepared by the method of the present invention.
[Fig. 4] The results of the tertiary screening. Comparison of 2D culture and 3D culture (spheroids prepared by using a plate having 96 wells with a U-shaped bottom section were used). PD0325901 was identified as a compound having high ability to inhibit spheroid formation.
[Fig. 5] The results of the tertiary screening. After the inoculation, the drug was promptly added (pre-treatment) or added one day afterward (post-treatment).
[Fig. 6] The effect of PD0325901 on peritoneal dissemination (fluorescence amount emitted from the alimentary canal extracted on the last evaluation day). Comparison with the vehicle group on the final day (Tukey-Kramer HSD test). The activity of the compound PD0325901 identified in the screening was confirmed in vivo by using mice.
[Fig. 7] The effect of PD0325901 on peritoneal dissemination (weight of the ascites collected on the last evaluation day). Comparison with the vehicle group on the final day (Tukey-Kramer HSD test). The activity of the compound PD0325901 identified in the screening was confirmed in vivo by using mice.

### Modes for Carrying out the Invention

Numerical value ranges indicated as "X to Y" include the values of X and Y as the maximum and minimum values, unless especially indicated. The expression "A and/or B" means at least one of A and B, unless especially indicated.

### [Aligned spheroids having equal sizes]

The present invention provides a culture of cells supported by a plate on which a plurality of wells are regularly arranged (well plate), wherein each well contains one or less spheroid. The culture contains aligned spheroids having equal sizes. The term spheroid means a cell mass formed by a large number of cells aggregated to form a three-dimensional structure. Cell masses usually have a shape close to a spherical shape. The expression that each well contains not more than one of spheroid means that the number of spheroid existing in each well is 1, or any spheroid does not exist in each well. For example, as for various controls provided for the purpose of confirming that experiments are appropriate, or in screening for an agent that inhibits spheroid formation, when spheroid is not formed, any spheroid may not be contained in the corresponding well.

Various kinds of cells that can form spheroids can be used for the present invention. The cell may be a cell relevant to a condition or disease in which formation of spheroids is involved. Specific examples of usable cells include undifferentiated cells and differentiated cells thereof that can form spheroids, such as cultured cells (cell strains), and stem cells (embryonic stem cells, cord blood-derived cells, undifferentiated mesenchymal stem cells, etc.). Examples of internal organs as the origin of the cells include liver, pancreas, large intestine, blood vessel, and nerve, and also include bone, fat tissue such as breast, ligament, tendon, tooth, auricle, nose, and so forth. The origin of the cells may be an animal (for example, laboratory animals such as mouse, rabbit, rat, guinea pig, dog, pig, goat, and cow or ox), or human. The cells may be genetically manipulated cells. The spheroid may not necessarily be formed as an aggregate of a single kind of cells, and may be formed from a plurality of kinds of cell species, so long as a spheroid is formed. The cells may be cancerous cells, or non-cancerous cells. The cell strain of cancerous cells may originate in large intestine cancer, prostate cancer, breast cancer, multiple myeloma, B cell lymphoma, malignant glioblastoma, renal cancer, liver cancer, prostate cancer, or parvicellular lung cancer.

In the present invention, the spheroids contained in the wells have equal sizes. Whether the spheroids have equal sizes or not can be judged by visual observation, or observation using an optical microscope. Individual spheroids formed by inoculating equal amounts of cells into wells usually have equal sizes. As for size of one spheroid, it consists of, for example, about 0.1 to 10.0 x 10³ cells, although it depends on cells and conditions used. Diameter of such a spheroid is typically 60 to 500 µm.

In the present invention, the spheroids formed are formed at intended positions, respectively, and are regularly arranged. The expression of regularly arranged means that they are arranged with one-dimensionally, two-dimensionally, or three-dimensionally equal intervals, and whether they are regularly arranged or not can be determined by visual observation, or observation using an optical microscope. When wells having the same shapes with a U-shaped section are used, spheroids are usually formed at the centers of the bottoms having a U-shaped section of the wells, and there can be obtained spheroids regularly arranged according to the arrangement of the wells. The state of the regularly arranged spheroids may be referred to as array state. The intervals of the spheroids can be variously adjusted by adjusting the intervals of the wells.

### [Method for preparing a plurality of regularly arranged spheroids having equal sizes]

Such a plurality of regularly arranged spheroids having equal sizes can be prepared by a preparation method comprising the following steps:
(1) the step of inoculating cells on a plate on which a plurality of wells are regularly arranged (well plate), wherein each of the wells has a lowly adsorptive bottom having a U-shaped section, at a density effective for formation of spheroid; and
(2) the step of culturing the cells in the plurality of the wells.

### Step (1)

The step (1) is a step of inoculating cells on a well plate at a density effective for formation of spheroid.

Well plate is also called microplate or microtiter plate, and refers to an experimental or test tool consisting of a plate having a large number of hollows (wells). The plate generally has a rectangular shape as the whole plate, the wells are arranged in lines at a ratio of 2:3, the number of the wells is 6, 24, 96, 384, 1536, or the like, and volume of each well is several µL to several mL. Although it is not particularly limited, a plate having 96, 384, or 1536 of wells can be especially preferably used for the present invention.

Material of the well plate is not particularly limited, so long as a material that does not have cytotoxicity and is suitable for cell culture is chosen, and there can be chosen one or a combination of two or more of acrylic resin, polylactic acid, polyglycolic acid, styrene resin, acrylic/styrene copolymer resin, polycarbonate resin, polyester resin, polyvinyl alcohol resin, ethylene/vinyl alcohol copolymer resin, thermoplastic elastomer, vinyl chloride resin, and silicone resin.

For the bottom of the well, a material showing a total luminous transmittance of 85% or higher and lower than 99% is preferably used, since optical analysis using fluorescence or based on light absorption is frequently used when such a screening as described later is carried out. The total luminous transmittance can be measured according to the Japanese Industrial Standard (JIS K7375).

For efficiently forming spheroids in the wells, internal surfaces of the wells (especially bottom surfaces of wells) are preferably lowly adsorptive. The expression of lowly adsorptive means a property of showing low adsorption or adhesion to proteins or cells. In order to make the wells lowly adsorptive, a material showing an instant water contact angle of, for example, 45° or smaller, preferably 40° or smaller, more preferably 20° or smaller, can be used, or the wells may be subjected to a surface treatment. Examples of the surface treatment include corona discharge treatment, plasma treatment, flame plasma treatment, UV treatment using a low-pressure mercury lamp, excimer UV treatment, laser treatment, electron beam treatment, and coating treatment. The instant water contact angle is measured by using a plate consisting of an objective material or a plate subjected to an objective surface treatment. The instant water contact angle means an angle between a solid surface and a tangent drawn at an end of surface of liquid contacting to the solid. As the contact angle, a value measured by the contact method using a water drop is used, and the instant water contact angle is the contact angle measured 1 minute after a water drop is dropped onto a solid surface. Relation between low adsorption (suppressed cell adhesion) and the instant water contact angle is described in, for example, Ikada, Y., Surface modification of polymers for medical applications, Biomaterials, 1994, vol. 15, No. 10, pp.725-736. In order to make the wells lowly adsorptive, a substance that suppresses cell adhesion may be coated. For example, a phospholipid/polymer composite, or poly(2-hydroxyethyl methacrylate) may be coated. Instant water contact angle of the internal surface of the well may be 45° or smaller, preferably in the range of 0 to 20°, from the viewpoint that the well is for containing the medium in the inside thereof.

As for shape of the bottom of the well of the well plate, the bottom is generally a flat bottom, bottom having a U-shaped section, bottom having a V-shaped section, or the like. In the present invention, the shape of the bottom of the well of the plate can be appropriately chosen depending on the cells with which spheroids are formed, but it is preferable to use one not having a flat bottom, and it is more preferable to use one having a bottom having a U-shaped section, in order to favorably form one spheroid at an intended position.

Although the well plate is not particularly limited, one comprising wells having a lowly adsorptive bottom and having a U-shaped section is preferred as described above.

The cells are inoculated at a density effective for spheroid formation. Such a density can be appropriately determined by those skilled in the art depending on the cells and well plate to be used, with performing a preliminary experiment and so forth, if needed, and when a well plate having 96 or more of lowly adsorptive wells with bottoms having a U-shaped section is used, the density may be, for example, 0.1 x 10⁴ cells/mL or higher, preferably 0.5 x 10⁴ cells/mL or higher, more preferably 1.0 x 10⁴ cells/mL or higher. The maximum density may be 3.0 x 10⁴ cells/mL or lower, preferably 3.0 x 10⁴ cells/mL, more preferably 3.0 x 10⁴ cells/mL or lower. When the cell density for the inoculation is low, it is assumed that desired spheroids may not be obtained by the culture in the following step (2) within a predetermined time. It is also assumed that spheroids having a sufficient size cannot be obtained. Spheroid of a very small size results in reduction of measurement sensitivity. When the cell density is high, it is thought that there may be cells that do not form spheroids. It is thought that, if there are a large number of cells that do not form spheroids, they may disturb the judgment based on whether there is spheroid formation or not.

### Step (2)

In the step (2), the inoculated cells are cultured in each well. This step is a step of making the inoculated cells form one spheroid using the space in the well. The culture can be performed by using a culture medium suitable for the cells to be used with conditions suitable for the cell culture (for example, 37°C, 5% CO₂, and 95% air) for 6 to 96 hours. When the spheroids or step of forming the spheroids according to the present invention is used for the screening described later or the like, the culture environment may be changed, for example, by adding an objective drug, before or during the culture step.

### [Use of spheroids]

It is known that a spheroid having a three-dimensional structure may more favorably reflect in vivo behaviors of many cells compared with the conventional two-dimensional culture. Therefore, researches of the interactions of tumor and immunocytes using spheroids, screening for drug development using spheroids, and so forth have been reported. Accordingly, the spheroids and the step for forming the spheroids provided by the present invention can be used for screening for a drug etc., various cell assays, monitoring of spheroid formation, elucidation of mechanism of a condition or disease in which formation of spheroid is involved, and so forth.

A plurality of spheroids having equal sizes and regularly arranged in a number of one in each well, and a step for forming such spheroids are provided by the present invention, and reliable screening can be performed by using such spheroids.

It is known that metabolic activity of spheroid differs depending on the size thereof. Therefore, spheroids of different sizes provide different metabolic activity values, and they cannot give highly accurate results. Further, it is also known that metabolic ability of extremely small spheroid is extremely low, and if such a spheroid is used, only a small amount of reaction metabolite is obtained, and measurement sensitivity may be reduced. A system consisting of spheroids of equal sizes larger than a certain size very scarcely exhibits such inconvenience. The Z' value, which is an index representing optimality of screening conditions and accuracy, is usually targeted to exceed 0.5 at the time of the optimization, and highly accurate screening can be performed with a Z' value exceeding 0.5 according to the present invention, which provides spheroids having equal sizes.

Since the spheroids are regularly arranged, it becomes easy to use automated robotic operation, and thus it becomes possible to simultaneously handle a large amount of samples.

Therefore, the present invention is extremely suitable for use in HTS, in which a compound having an intended activity is selected from a compound library constituted by an especially huge number of kinds of compounds by using an automated robot or the like.

### [Screening method]

The present invention provides a method for screening for a substance that acts on spheroid formation, which comprises the following steps (1) to (3):
(1) the step of inoculating cells on a plate on which a plurality of wells are regularly arranged (well plate), wherein each of the wells has a lowly adsorptive bottom having a U-shaped section, at a density effective for formation of spheroid, and culturing the cells in the plurality of the wells;
(2) the step of contacting the cells with a test substance; and
(3) the step of observing whether the cells contacted with the test substance form a spheroid or not, and evaluating action of the test substance on spheroid formation on the basis of the obtained observation result as an index.

### Screening step (1)

The screening step (1) is a step of inoculating cells on a well plate at a density effective for formation of spheroid, and culturing the cells in the plurality of the wells. As for the cells, well plate, inoculation conditions, and culture conditions to be used, the aforementioned explanations concerning the spheroids or the preparation method thereof can be referred to.

### Screening step (2)

The screening step (2) is a step of contacting the cells with a test substance. This screening step may be performed before the screening step (1), or may be performed in the middle of the screening step (1). When this step is performed before the screening step (1), this step can be performed by, for example, adding the test substance to a liquid in which the cells are suspended at the time of the inoculation of the cells. When this step is performed in the middle of the screening step (1), this step can be performed by, for example, inoculating the cells into the wells, culturing the cells for several hours if required, and then adding the test substance to the wells.

When screening is performed for the purpose of selecting an agent that inhibits formation of spheroids, the screening may be performed by using a substance of which anti-cancer action is known as a control, besides the test substance. Examples of such a substance include anti-cancer agents such as cisplatin, carboplatin, oxaliplatin, cyclophosphamide, ifosfamide, melphalan, busulfan, dacarbazine, ranimustine, nimustine, vincristine, irinotecan, docetaxel, paclitaxel, adriamycin, mitomycin, doxorubicin, epirubicin, daunorubicin, and bleomycin.

Although the contact time of the test substance and the cells is not particularly limited, it may be, for example, 6 hours or longer, or 12 hours or longer. Whether the spheroids are formed or not may be observed as described later for every 24 hours, 48 hours, 72 hours, 96 hours, or the like.

### Screening step (3)

The screening step (3) is a step of observing whether the cells contacted with the test substance form a spheroid or not, and evaluating action of the test substance on spheroid formation on the basis of the obtained observation result as an index.

Presence or absence of formation of spheroid can be observed by direct confirmation of the formation based on visual inspection, or may also be observed by quantification of an expression amount of a specific gene using a reporter gene, measurement of cell survival rate, or the like. Examples of the reporter include green fluorescent protein (GFP), Discosoma sp. red fluorescent protein (DsRed), chloramphenicol acetyltransferase (CAT), β-glucuronidase (GUS), and so forth. Examples of the method for measuring cell survival rate include ATP assay, MTT assay, intracellular glutathione assay, LDH assay, and so forth. These reporters and methods for measuring cell survival rate are well known to those skilled in the art.

The screening method of the present invention can be used for screening for a drug candidate compound for use in treatment (prophylactic treatment and therapeutic treatment) of a condition or disease in which formation of spheroid is involved, or a lead compound or seed compound thereof. The condition or disease in which formation of spheroid is involved may be peritoneal dissemination.

The screening method of the present invention can be performed in any stage of drug screening, and can be repeatedly performed. For example, it can be performed as phenotype assay using a high-content analysis, which is performed for one level of concentration of a test substance (primary screening), phenotype assay using a high-content analysis for confirming dose response of a test substance (secondary screening), and/or phenotype assay for narrowing down candidate compounds (tertiary screening).

Hereafter, the present invention will be specifically explained with reference to examples. However, the present invention is not limited by the following descriptions.

### Examples

### [Preparation of cells used for screening]

According to the following method, cryopreserved cells were thawed, subculured, and used for the screening described below.

### Thawing of cells

1. Take out a vial containing CT26 GFP⁺ cells (2.0 x 10⁶ cells/1 mL/ampoule) from a liquid nitrogen tank, and thaw the cells in a hot water bath at 37°C. The used cells can be prepared by introducing GFP into a parent strain purchased from ATCC using a SIV vector, and performing single cell cloning (refer to Non-patent documents 1 and 2 mentioned below).
2. Add the whole volume of the cell suspension in the vial to 10% FBS-RPMI 1640 (Dulbecco's modified eagle medium containing 10% fetal calf serum, 100 units/mL of penicillin, and 100 µg/mL of streptomycin), and then centrifuge the mixture at 4°C (100 g, 3 minutes).
3. Re-suspend the precipitates (cells) in 10 mL of 10% FBS-RPMI 1640, inoculate the cells on one 100-mm dish (BD Biosciences), and culture them under the conditions of 37°C, 5% CO₂, and 95% air.

### Subculture of CT26 GFP⁺

1. Proliferate CT26 GFP⁺ cells to a subconfluent or confluent state, and then subculture them.
2. Remove the culture medium by suction, and wash the cells with phosphate buffered saline (PBS, Invitrogen).
3. Remove PBS, then add a 0.05% trypsin solution [0.25% trypsin, 1 mmol/L EDTA•4Na (Invitrogen), diluted 5-fold with PBS], and allow the reaction under the conditions of 37°C, 5% CO₂, and 95% air until cells are separated from the dish.
4. Add 10% FBS-RPMI 1640 to suspend the cells, and collect them.
5. Perform centrifugation (400 g, 3 minutes) at 4°C, and then re-suspend the precipitates (cells) in 10% FBS-RPMI 1640.
6. Mix a part of the cell suspension with 0.4% trypan blue solution (Sigma-Aldrich), and count live cells under a phase contrast microscope by using a cell counting plate (OneCell Counter, OneCell Inc.).
7. Dilute the cell suspension with 10% FBS-RPMI 1640, and inoculate it at a density of 2.0 x 10⁵ cells/dish.
8. Discard the residual cells.

### [Primary screening and secondary screening]

### <Methods>

For a validated compound library obtained from the Drug Discovery Initiative, University of Tokyo (formerly Open Innovation Center for Drug Discovery, University of Tokyo) (library consisting of known active compounds and off-patent drugs, about 3,000 types), primary screening and secondary screening were performed by the following methods.

### Inoculation to 384 plate (for primary and secondary screenings)

1. Proliferate CT26 GFP⁺ cells to a subconfluent or confluent state, then remove the culture medium by suction, and then wash the cells with phosphate buffered saline (PBS, Invitrogen).
2. Remove PBS, then add a 0.05% trypsin solution [0.25% trypsin, 1 mmol/L EDTA•4Na (Invitrogen), diluted 5-fold with PBS], and allow the reaction under the conditions of 37°C, 5% CO₂, and 95% air until cells are separated from the dish.
3. Add 10% FBS-RPMI 1640 to suspend the cells, and collect them.
4. Perform centrifugation (400 g, 3 minutes) at 4°C, and then re-suspend the precipitates (cells) in 10% FBS-RPMI 1640.
5. Mix a part of the cell suspension with 0.4% trypan blue solution (Sigma-Aldrich), and count live cells under a phase contrast microscope by using a cell counting plate (OneCell Counter, OneCell Inc.).
6. In the case of 2D culture, dilute the cell suspension with 10% FBS-0.1% DMSO-RPMI 1640 to 3.0 x 10⁴ cells/mL, and inoculate the cells on a culture plate (ViewPlate-384, PerkinElmer) in a volume of 20 (µL/well using BIOMEK (registered trademark) NXP (BECKMAN COULTER).
7. In the case of 3D culture, dilute the cell suspension with 10% FBS-0.1% DMSO-RPMI 1640 to 5.0 x 10⁴ cells/mL, and inoculate the cells on a low adhesion plate (PrimeSurface (registered trademark) 384U plate, Sumitomo Bakelite Co., Ltd.) in a volume of 20 µL/well using BIOMEK (registered trademark) NXP.
8. Culture the inoculated cells under the conditions of 37°C, 5% CO₂, and 95% air.

Use 384 plate for both the primary screening and secondary screening.

An example of the setup screen of BIOMEK (registered trademark) NXP (BECKMAN COULTER) at the time of cell seeding and sucking on CellTiter (for both 2D and 3D) is shown below.

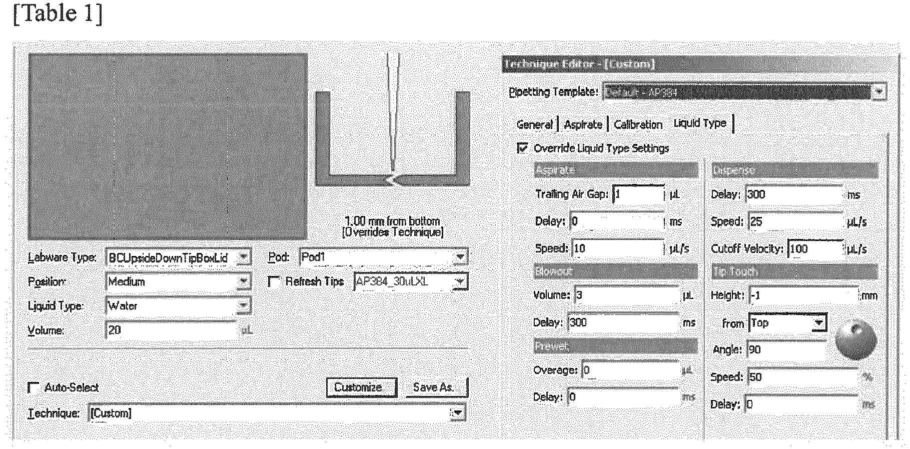

### Preparation of drug (for primary and secondary screenings)

1. Add 10% FBS-RPMI 1640 to a compound plate, BIOMEK (registered trademark) NXP, on which required amounts of compound are put into the wells to prepare a drug plate for addition.
2. Subsequently, add 10 µL of the solution of the drug plate to each well of the cell plate after the inoculation using BIOMEK (registered trademark) NXP, and perform pipetting.

Use DMSO at a concentration of 0.1 to 0.3%.

As for addition time of the drug, add the drug immediately after the inoculation, or one day after the inoculation.

In the primary screening, perform evaluation with a single (one dose for one compound per one well).

In the secondary screening, evaluate dose response of the compound with 4 doses (changeable as required).

An example of the setup screen of BIOMEK (registered trademark) NXP (BECKMAN COULTER) at the time of dilution of drug (addition of medium to the compound plate, for both 2D and 3D) is shown below.

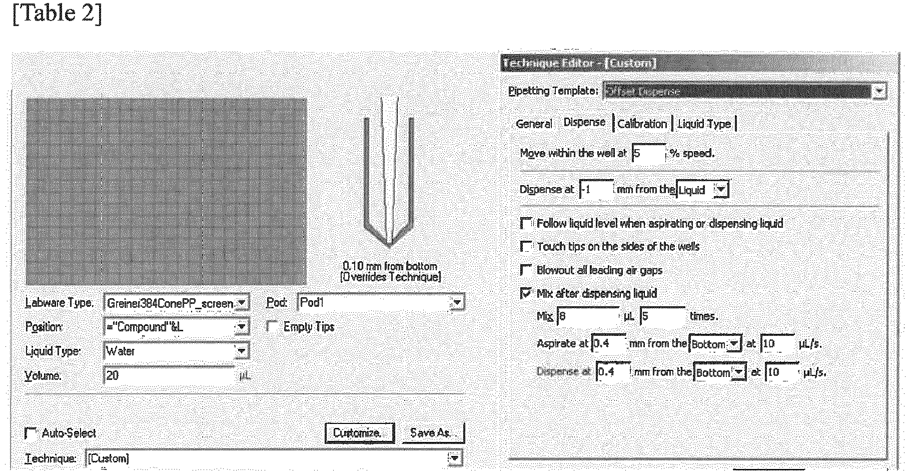

An example of the setup screen of BIOMEK (registered trademark) NXP (BECKMAN COULTER) at the time of addition of drug (2D) is shown below.

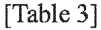

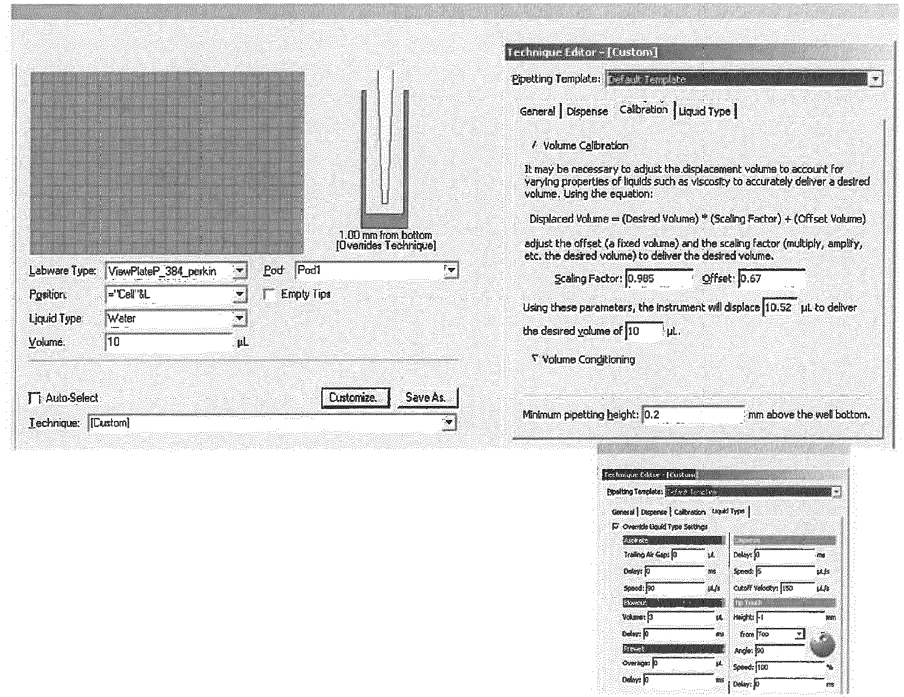

An example of the setup screen of BIOMEK (registered trademark) NXP (BECKMAN COULTER) at the time of addition of drug (3D) is shown below.

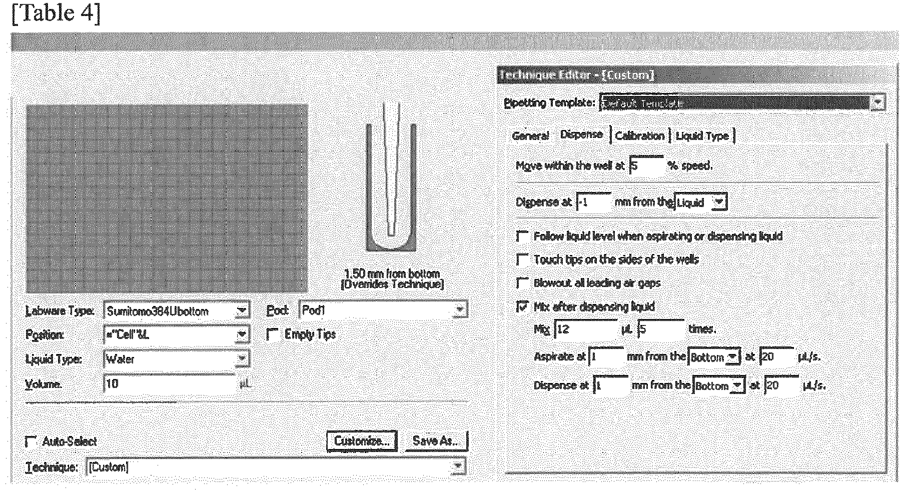

### Observation (primary and secondary screenings)

1. Observe morphology of the spheroids by 2 types of methods, in a bright field and with fluorescence, by using IN Cell Analyzer 2000 (GE Healthcare) 72 Hours after the treatment with the drug.
2. Evaluate the spheroids on the basis of the total fluorescence amount (density x area) obtained from GFP as an index.
3. In the primary screening, consider a well for which 50% or more of change is confirmed compared with DMSO-treated well as a HIT candidate.

Comprehensively determine final evaluation in consideration of the results of the ATP assay described in the following section.

### ATP assay (primary and secondary screenings)

1. On the final day of the evaluation, add CellTiter-Glo (registered trademark) 3D cell Viability Assay in the same volume as that of the culture volume by using BIOMEK (registered trademark) NXP, and 15 to 30 minutes thereafter, perform pipetting.
2. Subsequently, transfer 20 µL of the cell lysate to Black Plate as required, and measure luminescence with a luminometer (Enspire etc.).

### <Results>

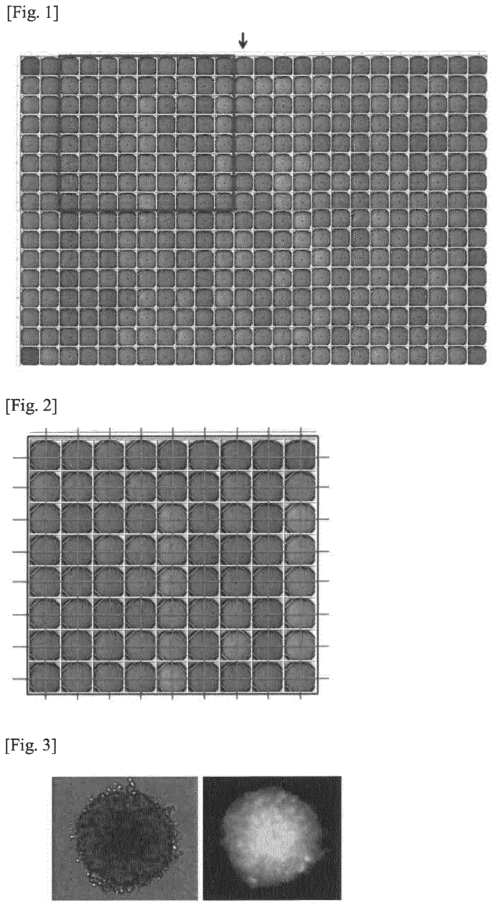

Photographs of the spheroids prepared by using a 384 plate are shown in Figs. 1 to 3. Those spheroids had equal sizes, and in each well, one spheroid locating at the center of the bottom having a U-shaped section was observed. The sizes of the spheroids in the wells were almost the same.

The CV value (coefficient of variation, calculation equation: CV (%) = Standard deviation (SD)/Average (Av), representing variations of volumes contained in the wells, values measured with a plate reader, and so forth, the CV value is generally preferably not larger than 10%), and the Z' factor (calculation equation: Z' = 1 - (3 x SD 100% + 3 x SD)/(Av 100% - Av 0%), Z' factor is a value serving as an index of quality of assay system, and is an important index representing accuracy, and a system giving a Z' factor of 0.5 or higher is generally preferred) were also calculated. As shown in the following table, both the CV value and Z'-factor were favorable. In order to take the influence of DMSO at the time of addition of the compound into consideration, examination was performed with two levels of concentration.

**[Table 5]**

| | DMSO Concentration | CV value | Z'-factor |
|---|---|---|---|
| 24h | 0.1% | 6.7 | 0.80 |
| | 1% | 7.0 | 0.79 |
| 48h | 0.1% | 6.2 | 0.81 |
| | 1% | 7.0 | 0.79 |
| 24h | 0.1% | 7.1 | 0.79 |
| | 1% | 5.8 | 0.83 |
| 95h | 0.1% | 5.5 | 0.84 |
| | 1% | 5.5 | 0.84 |

In the primary screening and secondary screening performed by using spheroids, 127 kinds of compounds and 8 kinds of compounds were retrieved, respectively.

### [Tertiary screening]

### <Methods>

Tertiary screening was performed by the following methods.

### Inoculation onto 96 plate

1. Proliferate CT26 GFP⁺ cells to a subconfluent or confluent state, then remove the culture medium by suction, and wash the cells with phosphate buffered saline (PBS, Invitrogen).
2. Remove PBS, then add a 0.05% trypsin solution [0.25% trypsin, 1 mmol/L EDTA•4Na (Invitrogen), diluted 5-fold with PBS], and allow the reaction under the conditions of 37°C, 5% CO₂, and 95% air until cells are separated from the dish.
3. Add 10% FBS-RPMI 1640 to suspend the cells, and collect them.
4. Perform centrifugation (400 g, 3 minutes) at room temperature, and then re-suspend the precipitates (cells) in 10% FBS-RPMI 1640.
5. Mix a part of the cell suspension with 0.4% trypan blue solution (Sigma-Aldrich), and count live cells under a phase contrast microscope by using a cell counting plate (OneCell Counter, OneCell Inc.).
6. In the case of 2D culture, dilute the cell suspension with 10% FBS-0.1% DMSO-RPMI 1640 to a density of 1.1 x 10⁴ cells/mL, and inoculate the cells onto a culture plate in a volume of 90 µL/well.
7. In the case of 3D culture, dilute the cell suspension with 10% FBS-0.1% DMSO-RPMI 1640 to a density of 2.2 x 10⁵ cells/mL, and inoculate the cells onto a low adhesion plate (Nunclon Sphera96, Thermo Scientific) in a volume of 90 µL/well with a pipet.
8. Culture the inoculated cells under the conditions of 37°C, 5% CO₂, and 95% air.

### Preparation of drug

1. Serially dilute a compound from the highest concentration of 20 µM at a common ratio of 1/10 for 7 doses (1% DMSO) with the medium.
2. Subsequently, add 10 µL of the serially diluted compound to the cell plate after the inoculation.
3. As for addition time of the drug, add the drug immediately after the inoculation (pre-treatment), or one day after the inoculation (post-treatment).

Perform the evaluation basically by a single experiment (n = 1).

### ATP assay

On the final day of the evaluation, add CellTiter-Glo (registered trademark) 3D cell Viability Assay in the same volume as that of the culture volume, and 15 to 30 minutes thereafter, perform pipetting. Subsequently, transfer 120 µL of the cell lysate to Black Plate as required, and measure luminescence with a luminometer (Enspire etc.).

### <Results>

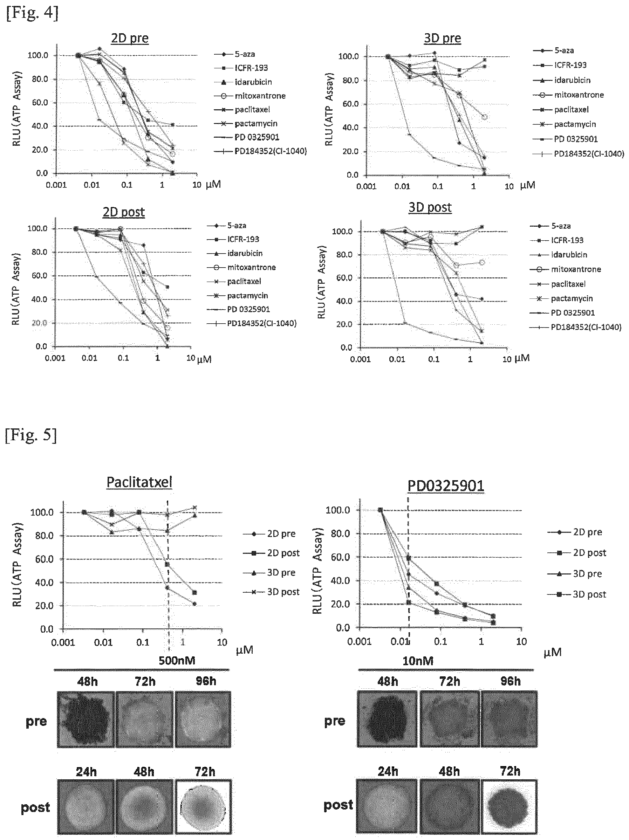

The results are shown in Figs. 4 and 5. As a result of the tertiary screening, PD0325901 was identified as a compound having high ability to inhibit spheroid formation.

### [In vivo test]

The activity of the compound PD0325901 identified by the screening was confirmed in vivo.

### <Methods>

### Animal

1. Use male BALB/c mice (henceforth referred to as mouse or mice, 6 to 8 weeks old at the time of use).
2. Put the mice in a plastic cage (W 136 mm x L 208 mm x H 115 mm) of which bottom is covered with a sterilized floor covering (Paper Clean, Japan SLC, Inc.) in a number of mice not larger than 5 mice per one cage until they are used for the experiments.
3. Allow the mice to ingest solid feed CRF-1 (Oriental Yeast Co., Ltd.) and tap water of water bottle ad libitum during the breeding period.
4. Perform individual identification of the mice by ear punching at the time of arrival of the mice (identification number starts from 01).

Thawing and subculture of cells are performed in the same manner as those described above.

### Intraperitoneal transfer

1. Proliferate CT26 GFP⁺ cells to a subconfluent or confluent state, then remove the culture medium by suction, and wash the cells with phosphate buffered saline (PBS, Invitrogen).
2. Remove PBS, then add a 0.05% trypsin solution [0.25% trypsin, 1 mmol/L EDTA•4Na (Invitrogen), diluted 5-fold with PBS], and allow the reaction under the conditions of 37°C, 5% CO₂, and 95% air until cells are separated from the dish.
3. Add 10% FBS-RPMI 1640 to suspend the cells, and collect them. Perform centrifugation (400 g, 3 minutes) at 4°C, and then re-suspend the precipitates (cells) in 10% FBS-RPMI 1640.
4. Mix a part of the cell suspension with 0.4% trypan blue solution (Sigma-Aldrich), count live cells under a phase contrast microscope by using a cell counting plate (OneCell Counter, OneCell Inc.), and prepare a cell suspension of 1.0 x 10⁷ cells/mL with HBSS.
5. Inject 0.2 mL of the cell suspension into the abdominal cavity of each mouse (2.0 x 10⁶ cells/body) by using a syringe with 26G hypodermic needle (TERUMO CORP.).

### Grouping

Put the mice in the descending order of the body weights on the day of the intraperitoneal injection (Day 0), assign random numbers to them, and divide them into required groups (stratified randomization method by weight).

Drug preparation: Prepare drug solution corresponding to optimal administration route.

Administration of drug solution: Appropriately choose or adjust administration scheme and dose depending on the drug.

Administration starting time: Start from Day 1 or Day 4.

Evaluation index and evaluation item:
Amount of disseminated nodules: GFP
Ascites weight: ascites
Survival time: day
Weight change: weight

### Evaluation method

### (1) Amount of disseminated nodules

On the last day of the evaluation, sacrifice the mice by cervical dislocation, open up the abdomen by midline incision, extract the alimentary canal from the duodenum to the rectum, and store it in ice-cooled PBS. Spread the extracted alimentary canal on a 100-mm dish, and obtain images of the total alimentary canal in a bright field and with fluorescence by using BZ9000. Convert the obtained images into numerical forms by using ImageJ, and use the results as amount of disseminated nodules.

### (2) Ascites weight

On the last day of the evaluation, sacrifice the mice by cervical dislocation, open up the abdomen by midline incision, and collect the ascites reserved in the abdominal cavity with absorbent cotton of which weight is measured beforehand. After the collection, measure the weight, and use the result as ascites weight.

### (3) Survival time

Record the dates on which deaths of the mice were confirmed during the test period.

### (4) Weight change

Record the body weights of the mice every 2 or 3 days during the test period.

### <Results>

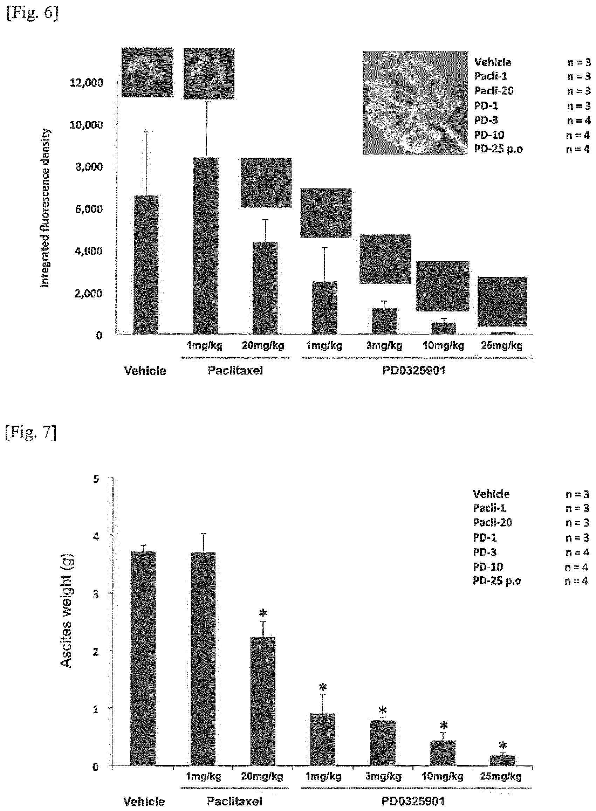

The results are shown in Figs. 6 and 7.

### [References cited in the section of Examples]

Non-patent document 1: Y Kasagi et al., Peritoneal Dissemination Requires an Sp1-Dependent CXCR4/CXCL12 Signaling Axis and Extracellular Matrix-Directed Spheroid Formation, Cancer Res., 76 (2) January 15, 2016, 347-357 (Published OnlineFirst January 7, 2016; DOI: 10.1158/0008-5472, CAN-15-1563)
Non-patent document 2: Y Ikeda et al., Simian immunodeficiency virus-based lentivirus vector for retinal gene transfer: a preclinical safety study in adult rats, Gene Therapy (2003) 10, 1161-1169

### Industrial Applicability

The aligned spheroids having equal sizes obtained by the present invention can be used for screening for a compound useful as an anticancer agent or the like, and they are also useful for elucidating pathology of a disease or condition involving spheroid formation such as peritoneal dissemination, and establishing prophylactic and therapeutic means for the same.

## Claims

1. A culture of cells supported by a plate on which a plurality of wells are regularly arranged (well plate), wherein each of the wells contains not more than one of spheroid.

2. The culture according to claim 1, wherein the spheroids in the wells have equal sizes.

3. The culture according to claim 1 or 2, wherein each of the wells has a lowly adsorptive bottom having a U-shaped section.

4. The culture according to any one of claims 1 to 3, wherein the well plate has 96, 384, or 1536 wells.

5. The culture according to any one of claims 1 to 4, wherein the cells are cancerous cells.

6. The culture according to any one of claims 1 to 5, which is for use in screening for a compound.

7. The culture according to claim 6, wherein the screening is for selecting a candidate compound of anticancer agent.

8. The culture according to any one of claims 1 to 5, which is for use in elucidation of a mechanism of a condition or disease in which formation of spheroids is involved.

9. The culture according to claim 8, wherein the condition or disease in which formation of spheroids is involved is peritoneal dissemination.

10. A method for screening for a substance that acts on spheroid formation, which comprises the following steps:
(1) the step of inoculating cells on a plate on which a plurality of wells are regularly arranged (well plate), wherein each of the wells has a lowly adsorptive bottom having a U-shaped section, at a density effective for formation of spheroid, and culturing the cells in the plurality of the wells;
(2) the step of contacting the cells with a test substance; and
(3) the step of observing whether the cells contacted with the test substance form a spheroid or not, and evaluating action of the test substance on spheroid formation on the basis of the obtained observation result as an index.

11. The screening method according to claim 10, wherein the cells are cancerous cells.

12. The screening method according to claim 10 or 11, wherein the density effective for formation of spheroid is 1.0 x 10⁴ to 3.0 x 10⁴ cells/mL.

13. A method for producing a plurality of regularly arranged spheroids having equal sizes, which comprises the following steps:
(1) the step of inoculating cells on a plate on which a plurality of wells are regularly arranged (well plate), wherein each of the wells has a lowly adsorptive bottom having a U-shaped section, at a density effective for formation of spheroid; and
(2) the step of culturing the cells in the plurality of the wells.

14. The production method according to claim 13, wherein the cells are cancerous cells.
